# EUROPEAN PATENT APPLICATION

(11) **EP 4 541 341 A1**
(43) Date of publication of application: **23.04.2025**
(21) Application number: 23204691.2
(22) Date of filing: 19.10.2023
(51) Int. Cl.: A61K 8/04, A61K 8/34, A61K 8/365, A61K 8/368, A61K 8/37, A61K 8/73, A61P 17/06, A61Q 19/00

(54) **METHOD OF PRODUCING A NANOEMULGEL COMPOSITION WITH CARE AND COSMETIC PROPERTIES AND A NANOEMULGEL COMPOSITION WITH CARE AND COSMETIC PROPERTIES**

(71) Applicant: WELLNANOPHARM Spolka z o.o., 30-129 Krakow (PL)
(72) Inventor: NOWAK, Krzysztof, 30-444 Libertow (PL); KULAWIK-PIORO, Agnieszka, 31-866 Krakow (PL); LASON, Elwira, 30-376 Krakow (PL); MALINOWSKA, Magdalena, 30-829 Krakow (PL); MIASTKOWSKA, Malgorzata, 30-438 Krakow (PL); SIKORA, Elzbieta, 31-990 Krakow (PL); SLIWA, Karolina, 30-389 Krakow (PL)
(74) Representative: Kacperski, Andrzej

(57) **Abstract**

Method is characterized in that a pre-emulsion is created as a carrier for the active substance in the form of a pentacyclic triterpenoid compound; this is done by dissolving the active substance until a homogeneous mixture is obtained; an emulsifier is then added, followed by adding a portion of the water phase; the prepared pre-emulsion undergoes a process of breaking down the particles of the active substance into nanoparticles equal to or smaller than 200 nm; a nanoemulgel is produced by introducing a separately prepared gelling matrix into the obtained nanoemulsion. The obtained nanoemulgel, as a base containing the active substance in the form of a pentacyclic triterpenoid compound with reduced nanoparticle size is combined with an agent containing the same repeated active substance in the form of a pentacyclic triterpenoid compound in a form not subjected to particle size reduction of the active substance, obtaining a skin care and cosmetic composition.

## Description

The subject of the invention is a method of producing a nanoemulgel composition with skin care and cosmetic properties, especially for psoriatic skin. The invention also relates to a nanoemulgel composition with skin care and cosmetic properties, for topical application.

The use of pentacyclic triterpene compounds, particularly ursolic acid, for cosmetic purposes is well known.

These compounds include the following derivatives:
- ursane type: ursolic acid, α-amyrin, uvaol,
- oleanane type: oleanolic acid, β-amyrin, erythrodiol,
- lupane type: lupeol, betulin, betulinic acid,
- taraxastane type: taraxasterol,
- taraxerane type: taraxerol (1-4).
- boswelliane type: β-boswellic acid, acetyl-β-boswellic acid, 11-keto-β-boswellic acid, acetyl-11-keto-β-boswellic acid.

Ursolic acid (3β-hydroxy-urs-12-en-28-oic acid) is a pentacyclic triterpenoid. Its name comes from bearberry (Arctostaphylos uva ursi), from which it was initially isolated (bearberry contains up to 0.75% ursolic acid). Ursolic acid usually occurs in plants along with its isomer - oleanolic acid (3β-hydroxy-olea-12-en-28-oic acid). Besides bearberry, ursolic acid is found in rosemary, sage, thyme, marjoram, lemon balm, hyssop, and lavender.

Among the most important properties of ursolic acid are: anti-inflammatory, antioxidant, anticancer, antiatherosclerotic and antiarrhythmic activities, as well as soothing irritations and improving skin condition. It has been shown that ursolic acid increases the activity of cells producing collagen and ceramides, by increasing the degree of hydration and elasticity of the skin. The anti-inflammatory action of ursolic acid is explained by improving the functioning of the body's defense mechanisms through inhibiting the activity of enzymes - lipoxygenase and cyclooxygenase, responsible for the occurrence of inflammatory reactions. Additionally, ursolic acid has been shown to have antimicrobial and antifungal properties and protects the skin against bacterial and fungal infections.

The fundamental problem with the use of ursolic acid is that triterpenic acids are characterized by very poor solubility, both in hydrophilic and hydrophobic substances, which affects the reduction of the physiological action of these compounds. Additionally, there is a problem with their precipitation from preparations during long-term storage. The solubility of triterpenic acids can be improved by esterification, but this does not solve the problems of precipitation (as indicated in the patent application EP 1 889 850 A1). A more effective solution is the appropriate choice of carrier for controlled application of this active substance.

The most popular form of substrate serving as carriers of active substances in cosmetic and dermatological products are emulsions. They represent a physicochemical form that effectively supports the maintenance of the skin's water-lipid balance and additionally has the ability to transport active substances, both polar and non-polar, into deeper layers of the skin.

Relatively new formulations for topical application on the skin delivering active substances are nanoemulsions. From a physicochemical point of view, they are liquid dispersion systems consisting of a water phase, an oil phase, and a surfactant, sometimes with the addition of a cosurfactant. Compared to classic cosmetic emulsions, nanoemulsions differ in the size of the inner phase droplets; in the case of nanoemulsions, these are values below 200 nm. Moreover, compared to macroemulsions, they also show other advantages, among which are resistance to phenomena destabilizing the system, such as sedimentation or creaming, ideal homogeneity of the final product, and a higher solubilization capacity of active substances, especially difficult-to-dissolve lipophilic substances, such as triterpenic acids. Literature data indicate that nanoemulsions, as carriers of the active substance, compared to classic emulsions or pure oil base, are a more effective form of delivery to the skin. Considering the rheological properties of nanoemulsions, it should be noted that they are liquid dispersion systems with low viscosity, which allows for application of the preparation in the form of a spray, but may pose a problem in local application to the skin.

Hydrogels are a known form of active substance delivery systems, produced by using natural biopolymers, such as dextran, dextrin, chitosan, poly-y glutamic acid (y-PGA), hyaluronic acid, or alginates, or synthetic biodegradable and biocompatible polymers, e.g., poly (methyl methacrylate) (PMMA), polylactide (PLA), polyglycolide (PGA), or poly d,l-lactic-glycolic acid (PLGA). Despite many advantages, their disadvantage is relatively low mechanical stability.

Combining the properties of hydrogel and nanoemulsion into a nanoemulgel form allows obtaining a more universal, biocompatible active substance delivery system (both lipophilic and hydrophilic). As a result, we obtain a carrier that forms a capsule for various types of bioactive compounds (including difficult-to-dissolve, hydrophobic ones, such as triterpenic acids) and biopolymers. It should be emphasized that nanogels formed by appropriate cross-linking show high stability and flexibility.

In the patent application EP 1 889 850 A1, the structure of a new derivative of ursolic acid (ursolic acid phosphate) obtained by phosphorylation of at least one hydroxyl group of ursolic acid or its salts was disclosed. The phosphate derivative of the acid is characterized by much greater solubility in solvents such as ethanol, phosphate buffer, or their mixture. The description also disclosed a formulation of a cosmetic preparation in the form of an anti-inflammatory lotion and a melanin production inhibiting lotion containing from 0.3 to 1% solution of ursolic acid phosphate.

In the patent application US 2013/0017239A1, formulations of SLN and NLC were presented as potential carriers also for active substances belonging to metalloproteinase inhibitors such as ursolic acid or isoflavones. Meanwhile, in another patent application US 2006/0257386A1, a formulation of a cosmetic preparation aimed at preventing skin aging was described. The preparation contains an extract of rosemary in the amount of 0.01 to 0.5%, one of the main components of which is ursolic acid.

In the patent application KR 20060126247A, a solution is presented for a microemulsion for external use, which contains a mixture of benzyl alcohol and ethanol, and ursolic acid as the active substance. The patent US 4 857 554 describes the composition of an ointment intended for treating symptoms of psoriasis, containing ursolic and oleanolic acid in a weight ratio of 3:1, dissolved in a mixture of petroleum jelly and lanolin.

The invention solves the above-mentioned problem of the very poor solubility of triterpenic compounds. The solution involves that the particles of the active substance - pentacyclic triterpenoid compounds - contained in the pre-emulsion are subjected to a process of breaking down to the size of nanoparticles equal to or smaller than 200 nm through the process of ultrasonification - the action of ultrasound of a given power and amplitude. At the same time, such minimized particles of the active substance are used both in the base composition and by combining it with other preparations - in user compositions based on it, intended for various applications.

The method of producing a nanomulgel composition according to the invention involves the following steps:
a pre-emulsion is created as a carrier for the active substance in the form of a pentacyclic triterpenoid compound; this is done by dissolving the active substance in an amount of 0.1 to 0.5% by mass in oily emollients in an amount of 10 to 20% by mass at a temperature of 70-80 °C until a homogeneous mixture is obtained; an emulsifier is then added in an amount of 2 to 4% by mass, followed by adding a portion of the water phase with a preservative in an amount of 2.5 to 4.5% by mass, maintaining the temperature at 70-80 °C and mechanically stirring at a speed of 300 to 500 rpm during the pre-emulsification for 5 to 15 minutes at a temperature of 20-25 °C;
in the next stage, the prepared pre-emulsion undergoes a process of breaking down the particles of the active substance - the pentacyclic triterpenoid compound, into nanoparticles equal to or smaller than 200 nm; this is achieved by the application of ultrasound with a power of 20 to 40 W and an amplitude of 50 to 90 µm for a duration of 60 to 120 seconds, resulting in the formation of a nanoemulsion;
in the final stage, a nanoemulgel is produced by introducing a separately prepared gelling matrix into the obtained nanoemulsion; this matrix consists of a portion of the water phase, dispersed with a gelling agent in an amount of 1 to 2% by mass, heating the system to a temperature of 40-50°C, adding a pH regulator q.s. to achieve a pH of 6, and simultaneously mechanically stirring at a speed of 400 to 600 rpm, until a homogeneous nanoemulgel system is obtained.

The active substance is a pentacyclic triterpenoid compound selected from a group that includes ursolic acid, oleanolic acid, betulinic acid, betulin, boswellic acids (β-boswellic acid, acetyl-β-boswellic acid, 11-keto-β-boswellic acid, acetyl 11-keto-β-boswellic acid), α- and β-amyrin, and lupeol. The active substance, preferably ursolic acid or oleanolic acid, constitutes 0.1 to 0.5% by mass.

As oily emollients in an amount of 10 to 20% by mass, caprylic and capric triglycerides are used.

As an emulsifier in an amount of 2 to 4% by mass, polyglycerol-6 esters of olive oil combined with sodium stearoyl lactylate and cetearyl alcohol are used.

As a preservative in an amount of 2 to 4% by mass, an aqueous solution of glycerin, sodium levulinate, sodium anisate, and sodium benzoate is used.

Citric acid is used as a pH regulator q.s.

As a gelling agent in an amount of 1 to 2% by mass, sodium hyaluronate is used.

The obtained nanoemulgel, as a base containing the active substance in the form of a pentacyclic triterpenoid compound in an amount of 0.1 to 0.5% by mass with reduced nanoparticle size equal to or smaller than 200 nm, is combined in a mass ratio ranging from 1:9% to 9:1% or from 5:95% to 95:5% with an agent containing the same repeated active substance in the form of a pentacyclic triterpenoid compound in an amount of 0.1 to 1.0% by mass but in a form not subjected to particle size reduction of the active substance, obtaining a nourishing and cosmetic composition.

The obtained nanoemulgel, as a base containing the active substance in the form of a pentacyclic triterpenoid compound in an amount of 0.1 to 0.5% by mass with reduced nanoparticle size equal to or smaller than 200 nm, is combined in a mass ratio ranging from 10:90 to 90:10 with an agent in the form of a cream, serum, balm, containing the same repeated active substance in the form of a pentacyclic triterpenoid compound in an amount of 0.1 to 1.0% by mass in a form not subjected to particle size reduction of the active substance, obtaining a nourishing and cosmetic composition.

In another variant, the obtained nanoemulgel, as a base containing the active substance in the form of a pentacyclic triterpenoid compound in an amount of 0.1 to 0.5% by mass with reduced nanoparticle size equal to or smaller than 200 nm, is combined in a mass ratio ranging from 5:95 to 40:60 with an agent in the form of an oleogel, a scalp preparation, containing the same repeated active substance in the form of a pentacyclic triterpenoid compound in an amount of 0.1 to 1.0% by mass in a form not subjected to particle size reduction of the active substance, obtaining a nourishing and cosmetic composition.

The composition according to the invention, with skin care and cosmetic properties for topical application to the skin, especially for psoriatic skin, takes the form of a nanoemulgel and contains:
- as the active substance, a pentacyclic triterpenoid compound in an amount of 0.1 to 0.5% by mass, in the form of nanoparticles with a particle size reduced to equal to or smaller than 200 nm, selected from a group that includes ursolic acid, oleanolic acid, betulinic acid, betulin, boswellic acids (β-boswellic acid, acetyl-β-boswellic acid, 11-keto-β-boswellic acid, acetyl 11-keto-β-boswellic acid), α- and β-amyrin, lupeol
- oily emollients in an amount of 10 to 20% by mass
- emulsifier in an amount of 2 to 4% by mass
- preservative in an amount of 2.5 to 4.5% by mass
- pH regulator q.s.
- gelling agent in an amount of 1 to 2% by mass
- water up to 100%.

Ursolic or oleanolic acid in an amount of 0.1-0.5% serves as the active substance of the composition.

The oily emollients of the composition, in the amount of 10 to 20% by mass, consist of caprylic and capric triglycerides. The emulsifier, in an amount of 2 to 4% by mass, consists of polyglycerol-6 esters of olive oil along with sodium stearoyl lactylate and cetearyl alcohol.

The preservative, in an amount of 2.5 to 4.5% by mass, is an aqueous solution of glycerin, sodium levulinate, sodium anisate, and sodium benzoate.

The pH regulator q.s. is citric acid.

The gelling agent, in an amount of 1 to 2% by mass, is sodium hyaluronate.

The composition, as a base nanoemulgel, is combined in a mass ratio ranging from 10:90 to 90:10 with an agent in the form of a cream, serum, or balm, containing the same repeated active substance in the form of triterpenic acid in an amount of 0.1 to 1.0% by mass in a form not subjected to particle size reduction.

In another variant, the composition, as a base nanoemulgel, is combined in a mass ratio ranging from 5:95 to 40:60 with an agent in the form of an oleogel, scalp preparation, containing the same repeated active substance in the form of triterpenic acid in an amount of 0.1 to 1.0% by mass in a form not subjected to particle size reduction.

The advantage of this solution is the production of a preparation with the properties of a stable nanogel, based on an oil-in-water type nanoemulsion, with good sensory properties, containing a relatively small amount of surfactant, which belongs to the group of mild emulsifiers. The oil phase acts as a carrier for lipophilic substances, a transdermal penetration promoter, and improves the oxidative stability of preparations. Ursolic acid, for instance, exhibits antioxidative, anti-inflammatory, melanin production inhibiting, anti-aging, anticancer properties, and soothes symptoms of psoriasis. Therefore, the composition according to the invention and products made on its basis can be used to support skin regeneration and alleviate psoriasis symptoms.

The physicochemical form of the preparation as a nanoemulgel ensures increased transdermal penetration of the active substance due to the high degree of dispersion of the internal phase particles (the average size of the oil phase particles of the nanoemulsion is smaller than 200 nm), in which the active substance is contained.

Changing the composition within the limits anticipated by this solution allows obtaining a product with properties ranging from nourishing to cosmetic. Using appropriately large amounts of the active ingredient consequently leads to a preparation that improves skin hydration, can be used to prevent aging of the skin, and in alleviating symptoms of psoriasis. All products are a hybrid combination of a nanoemulgel and preparations such as creams, serums, and balms.

The solution according to the invention is presented in execution examples, for which ursolic acid was chosen as the active substance as an example of a pentacyclic triterpenoid. Instead of ursolic acid, it is possible to use, in the same range, another pentacyclic triterpenoid compound, selected from the group described above.

### Example 1

### Preparation of nanoemulgel with active substance

To obtain a nanoemulgel, the gelling agent - sodium hyaluronate 1.5-2. MDa 1% by mass - was first weighed and then dispersed in 30% by mass of the water phase to create a hydrogel matrix (Phase A), leaving it for one day.

Next, the oil phase (phase B) was prepared by adding the active substance - ursolic acid 0.1% by mass to the emollient - caprylic/capric triglycerides 10% by mass, and heated to dissolve the acid at a temperature of 70-80 degrees C. Separately, at the same temperature, the emulsifier in the form of polyglycerol-6 esters of olive oil, with additional 4% by mass, was dissolved and then added to the homogenous mixture of ursolic acid and emollient.

The ingredients of the water phase C, water up to 100% by mass, and the mixture of levulinic and anisic acid salts 2% by mass, were also heated to a temperature of 70 degrees C. Maintaining this temperature, the oil phase B and the water phase C were combined. The whole was mixed (mechanical stirrer RW 20 digital IKA; V= 300 rpm) for 10 minutes to obtain a homogenous consistency, thus creating a pre-emulsion.

Next, the pre-emulsion underwent a process of breaking down the particles of the active substance - ursolic acid - to nanoparticle size equal to or smaller than 200 nm through the process of ultrasonification -the application of ultrasound, using an ultrasonicator with a probe, applying a power of 20 W, at an amplitude of 50 to 100 µm for a duration of 120 seconds. The process was conducted using the UP200Ht Hielscher device. The system was supplied with energy from 60 Kj/kg to 85 Kj/kg of mass.

Then, the obtained nanoemulsion was combined with the hydrogel matrix, previously heated to a temperature of 40 degrees C, mixing using a mechanical stirrer at a stirrer speed of 500 rpm to obtain a homogenous system of the appropriate consistency. In the meantime, a 10% solution of citric acid heated to 40 degrees was added to lower the pH from 7 to 6.

A nanoemulgel with a wide spectrum of activity was obtained, with the following composition a listed in Table 1

**Table 1. Formula of nanoemulgel with active substance**

| **Phase** | **Function/properties** | **Ingredient** | **INCI name** | **Composition [%] by weight** |
|---|---|---|---|---|
| A gelling matrix: | water | water | Aqua | 30,0 |
| | gelatinizer | gelling agent: Sodium Hyaluronate (1.5-2.0 MDa) | Sodium Hyaluronate | 1,0 |
| B oil phase | emollients | Caprylic and Capric Triglycerides | Caprylic/Capric Triglyceride | 10 |
| | emulsifier | Polyglycerol-6 Esters of Olive Oil, Sodium Stearoyl Lactylate, Cetearyl Alcohol | Olive Oil Polyglyceryl-6 Esters, Sodium Stearoyl Lactylate, Cetearyl Alcohol | 4,0 |
| | active substance | Ursolic Acid | Ursolic Acid | 0,1 |
| C water phase | water | water | Aqua | up to 100 |
| | preservative | Sodium Levulinate, Sodium Anisate combined with Water and Glycerin | Aqua (and) Glycerin (and) Sodium Levulinate (and) Sodium Anisate | 2,0 |
| | preservative | Sodium Benzoate | Sodium Benzoate | 0,5 |
| | pH regulator | Citric Acid (10% solution) | Citric Acid | q. s. |

### Example II

### Preparation of nanoemulgel with active substance

To produce a nanoemulgel, the gelling agent - sodium hyaluronate 1.5-2. MDa 2% by mass - was first weighed and then dispersed in 30% by mass of the water phase to create a hydrogel matrix (Phase A), leaving it for one day.

Next, the oil phase (Phase B) was prepared by adding the active substance - ursolic acid 0.5% by mass to the emollient - caprylic/capric triglycerides 20% by mass, and heated to dissolve the acid at a temperature of 70-80 degrees C. Separately, at the same temperature, the emulsifier in the form of polyglycerol-6 esters of olive oil, with additions, 2% by mass, was dissolved and then added to the homogenous mixture of ursolic acid and emollient.

The ingredients of the water phase C, water up to 100% by mass, and the mixture of levulinic and anisic acid salts 4% by mass, were also heated to a temperature of 70 degrees C. Maintaining this temperature, the oil phase B and the water phase C were combined. The whole was mixed (mechanical stirrer RW 20 digital IKA; V= 300 rpm) for 10 minutes to obtain a homogenous consistency, thus creating a pre-emulsion.

Then, the pre-emulsion underwent a process of breaking down the particles of the active substance - ursolic acid - to nanoparticle size equal to or smaller than 200 nm through the process of ultrasonification -the application of ultrasound, using an ultrasonicator with a probe, applying a power of 40 W, at an amplitude of 50 to 100 µm for a duration of 60 seconds. The process was conducted using the UP200Ht Hielscher device. The system was supplied with energy from 60 Kj/kg to 85 Kj/kg of mass.

Then, the obtained nanoemulsion was combined with the hydrogel matrix, previously heated to a temperature of 40 degrees C, mixing using a mechanical stirrer at a stirrer speed of 500 rpm to obtain a homogenous system of the appropriate consistency. In the meantime, a 10% solution of citric acid heated to 40 degrees was added to lower the pH from 7 to 6.

A nanoemulgel with a wide spectrum of activity was obtained, with the following composition as listed in Table 2

**Table 2. Formula of nanoemulgel with active substance**

| **Phase** | **Function/properties** | **Ingredient** | **INCI name** | **Composition [%] by weight** |
|---|---|---|---|---|
| A Gelling Matrix | Water | Water | Aqua | 30,0 |
| | Gelatinizer | Gelling Agent: Sodium Hyaluronate (1.5-2.0 MDa) | Sodium Hyaluronate | 2,0 |
| B Oil Phase | Emollients | Caprylic and Capric Triglycerides | Caprylic/Capric Triglyceride | 20 |
| | Emulsifier | Polyglycerol-6 Esters of Olive Oil, Sodium Stearoyl Lactylate, Cetearyl Alcohol | Olive Oil Polyglyceryl-6 Esters, Sodium Stearoyl Lactylate, Cetearyl Alcohol | 2,0 |
| | Active Substance | Ursolic Acid | Ursolic Acid | 0,5 |
| C Water Phase | Water | Water | Aqua | Up to 100 |
| | Preservative | Sodium levulinate and sodium anisate with water and glycerin | Aqua (and) Glycerin (and) Sodium Levulinate (and) Sodium Anisate | 4 |
| | Preservative | Sodium Benzoate | Sodium Benzoate | 0,5 |
| | pH Regulator | Citric Acid (10% solution) | Citric Acid | q. s. |

The obtained nanoemulgel with the active substance in the form of ursolic acid or another pentacyclic triterpenoid compound, such as oleanolic acid, serves as a base for producing other products with the same active substance, with properties ranging from nurturing to cosmetic.

Products containing a repeated (double) dose of the active substance include:
- in the base, which is the nanoemulgel in an amount of 0.1 to 0.5% by mass with reduced nanoparticle size equal to or smaller than 200 nm
- in the product subjected to mixing with the base, in an amount of 0.1 to 1% by mass without nanoparticle size reduction.

### Example III

### Cosmetic facial cream with active substance

Combining cream - with nanoemulgel as a base containing the active substance in the form of ursolic acid in an amount of 0.1% by mass, with reduced nanoparticle size to 100 nm.

| **Name of the preparation** | **% by weight of** nanoemulgel | **% by weight of cream** |
|---|---|---|
| Cream-nanoemulgel | 10 | 90 |
| Cream- nanoemulgel | 20 | 80 |
| Cream- nanoemulgel | 30 | 70 |
| Cream- nanoemulgel | 40 | 60 |
| Cream- nanoemulgel | 50 | 50 |
| Cream- nanoemulgel | 60 | 40 |
| Cream- nanoemulgel | 70 | 30 |
| Cream- nanoemulgel | 80 | 20 |
| Cream- nanoemulgel | 90 | 10 |

### Procedure for obtaining:

Emulsion preparation based on nanoemulgel was obtained through homogenization using a high-speed stirrer. Firstly, a previously developed cream with ursolic acid at 1%, without particle size reduction of the active substance, was obtained. Then, the obtained cream was combined in a mass ratio presented in the table above with nanoemulgel containing ursolic acid at 0.1%, with reduced nanoparticle size to 100 nm, at a temperature of 40°C at 500 rpm/min and mixed until room temperature was reached.

### Example IV

### Serum with active substance

Combining serum - with nanoemulgel as a base containing the active substance in the form of ursolic acid at 0.3% by mass, with reduced nanoparticle size to 150 nm.

| **Name of preparation** | **% by weight of** nanoemulgel | **% by weight of serum** |
|---|---|---|
| Serum- nanoemulgel | 10 | 90 |
| Serum- nanoemulgel | 20 | 80 |
| Serum- nanoemulgel | 30 | 70 |
| Serum- nanoemulgel | 40 | 60 |
| Serum- nanoemulgel | 50 | 50 |
| Serum- nanoemulgel | 60 | 40 |
| Serum- nanoemulgel | 70 | 30 |
| Serum- nanoemulgel | 80 | 20 |
| Serum- nanoemulgel | 90 | 10 |

### Procedure for obtaining:

The emulsion preparation based on nanoemulgel was obtained through homogenization using a high-speed stirrer. Firstly, a previously developed serum with ursolic acid at 0.5%, without particle size reduction of the active substance, was obtained. Then, the obtained serum was combined in a mass ratio presented in the table above with nanoemulgel containing ursolic acid at 0.3%, with reduced nanoparticle size to 150 nm, at a temperature of 40°C at 300 rpm/min and mixed until room temperature was reached.

### Example V

### Body balm with active substance

Combining balm - with nanoemulgel as a base containing the active substance in the form of ursolic acid at 0.5% by mass, with reduced nanoparticle size to 180 nm

| **Name of preparation** | **% by weight of** nanoemulgel | **% by weight of balm** |
|---|---|---|
| **Balm- nanoemulgel** | **10** | **90** |
| Balm- nanoemulgel | 20 | 80 |
| Balm- nanoemulgel | 30 | 70 |
| Balm- nanoemulgel | 40 | 60 |
| Balm- nanoemulgel | 50 | 50 |
| Balm- nanoemulgel | 60 | 40 |
| Balm- nanoemulgel | 70 | 30 |
| Balm- nanoemulgel | 80 | 20 |
| Balm- nanoemulgel | 90 | 10 |

### Procedure for obtaining:

The emulsion preparation based on nanoemulgel was obtained through homogenization using a high-speed stirrer. Firstly, a previously developed balm with ursolic acid at 0.1%, without particle size reduction of the active substance, was obtained. Then, the obtained balm was combined in a mass ratio presented in the table above with nanoemulgel containing ursolic acid at 0.5%, with reduced nanoparticle size to 180 nm, at a temperature of 40°C at 500 rpm/min and mixed until room temperature was reached.

### Example VI

### Bigel with active substance

Combining oleogel - with nanoemulgel as a base containing the active substance in the form of ursolic acid at 0.3% by mass, with reduced nanoparticle size to 200 nm

| **Name of preparation** | **% by weight of** nanoemulgel | **% by weight of serum** |
|---|---|---|
| Oleogel- nanoemulgel | 5 | 95 |
| Oleogel- nanoemulgel | 40 | 60 |

### Procedure for obtaining:

An emulsion preparation based on nanoemulgel was obtained through homogenization using a high-speed stirrer. Initially, a previously developed oleogel based on oil and silica, with ursolic acid at 0.1%, without particle size reduction of the active substance, was obtained. Then, the obtained oleogel was combined in a mass ratio presented in the table above with nanoemulgel containing ursolic acid at 0.5%, with reduced nanoparticle size to 200 nm, at room temperature, gradually increasing the stirring speed (the oleogel increases its viscosity after adding the nanoemulgel), at 500 rpm/min and mixed until a homogeneous mixture was obtained.

### Example VII

### Scalp care preparation with active substance

Combining scalp care preparation - with nanoemulgel as a base containing the active substance in the form of ursolic acid at 0.5% by mass, with reduced nanoparticle size to 200 nm

| **Name of preparation** | **% by weight of nanoemulsion** | **% by weight of serum** |
|---|---|---|
| Preparation- nanoemulgel | 5 | 95 |
| Preparation- nanoemulgel | 40 | 60 |

### Procedure for obtaining:

A nanoemulsion-based scalp formulation was obtained by homogenization using a high-speed stirrer. The previously developed formulation was first obtained, with ursolic acid at 0.5%, without reducing the particle size of the active substance. Then, the resulting formulation was combined at the mass ratio shown in the table above with a nanoemulsion containing ursolic acid in an amount of 0.5%, with the nanoparticle size reduced to 200 nm, at ambient temperature by gradually increasing the stirrer speed at 150 rpm to avoid foaming of the system, and stirred until a homogeneous mixture was obtained.

### Stability testing of products

The stability of cosmetic products was analyzed using a Turbiscan LabCooler device from Formulation, enabling characterization of physicochemical phenomena of dispersions, including particle migration, change in particle size. The measuring head of the device (light source with two synchronized transmission and backscattering sensors) scans the preparation moving from the bottom to the top of the vial, collecting data every 40µm along the entire height of the sample (up to 50mm).

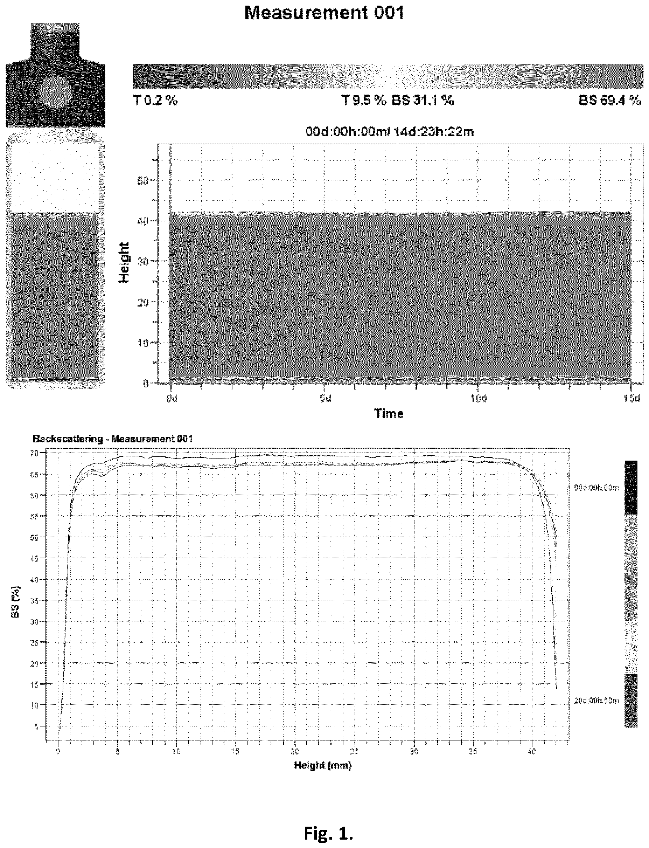

The results of the studies were presented in a chart generated by the software of the Turbiscan LAB version 2.0.0.19 (fig. 1).

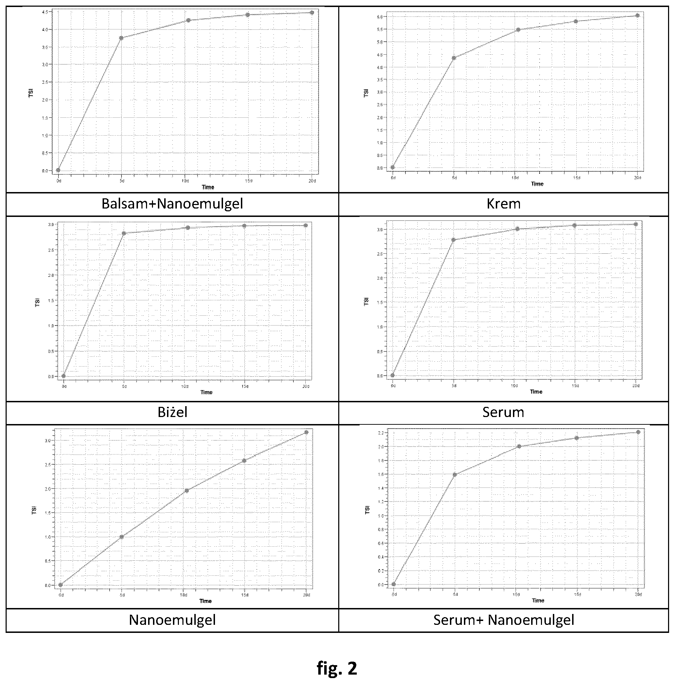

The results were interpreted based on the Turbiscan Stability Index (TSI). The higher the TSI value, the less stable the product (fig. 2). The cosmetic product serum + nanoemulgel had the lowest TSI value of about 2.2, indicating its most favorable stability among the tested products. Next were the serum, nanoemulgel, and bigel. Their maximum TSI values were similar and about 3. The highest TSI values were for the cream, body balm, and body balm + nanoemulgel. TSI values for these products ranged from 4.5 to 6.0, but such values also confirm the stability of the tested preparations. Preparations with a TSI >10 are considered unstable.

### Consistency testing

Consistency tests were performed using a Brookfield CT3 texture analyzer. A spherical nylon probe TA43, 14g (probe immersion depth of 10 mm at a constant head travel speed of 0.5 mm/s) was used. The results were recorded by the TexturePro CT software. In the Texture Profile Analysis (TPA), hardness was determined, measured as the mass needed to press the probe to a depth of 10 mm (maximum force recorded during the 1st test cycle) and the adhesion force measured as the mass required to pull the probe out.

**Table 3 Results of consistency tests**

| **Product** | **Hardness [g]** | | **Adhesion strength [g]** | |
|---|---|---|---|---|
| | | **Average** | | **Average** |
| Balm+Nanoemulsion | 3.5 | 4.2 | -2.0 | -2 |
| | 4.5 | | -2.0 | |
| | 4.5 | | -2.0 | |
| Balm | 21.00 | 21.5 | -7.0 | -7.2 |
| | 23.0 | | -7.0 | |
| | 20.5 | | -7.5 | |
| Bigel | 237.0 | 233.8 | -65.0 | -66.2 |
| | 235.5 | | -65.5 | |
| | 229.0 | | -68.0 | |
| Cream | 17.0 | 15.8 | -6.0 | -6.2 |
| | 16.5 | | -6.5 | |
| | 14.0 | | -6.0 | |
| Nanoemulgel | 49.5 | 49.8 | -15.0 | -15.8 |
| | 50.0 | | -16.5 | |
| | 50.0 | | -16.0 | |
| Serum+Nanoemulgel | 6.5 | 6.7 | -3.5 | -3.5 |
| | 7.0 | | -3.5 | |
| | 6.5 | | -3.5 | |
| Serum | 7.5 | 7.8 | -4.0 | -4.5 |
| | 8.0 | | -4.5 | |
| | 8.0 | | -5.0 | |

The texture analysis of the preparations, through measuring adhesion force and hardness, allowed for an objective estimation and comparison of those cosmetic characteristics that are usually subjectively assessed by users through sensory perception. Generally, it is accepted that higher hardness values indicate a more compact, denser consistency of the cosmetic product. These results often correlate with dynamic viscosity values. Furthermore, in the interpretation, one can refer to the application of the product from the packaging onto the skin.

Negative values indicate the adhesion force, which reflects the cosmetic's stickiness to the skin. The higher the values (closer to 0), the lesser the adhesion force.

### Microbiological tests

Preliminary microbiological tests and preservation tests were also conducted. The microbiological purity of selected 4 preparations (nanoemulgel, cream, balm, biogel) was tested in accordance with standards: PN-EN ISO 21149:2009, PN-EN ISO 162012:2011, PN-EN ISO 22718:2010, PN-EN ISO 21150:2010, and PN-EN ISO 18416:2009. Preservation tests were conducted according to the standard PN-EN ISO 11930:2012, using standard strains: Pseudomonas aeruginosa ATCC 9027, Staphylococcus aureus ATCC 6538, Escherichia coli ATCC 11229, Candida albicans ATCC 10231, Aspergillus brasilensis ATCC. The test involved, for each microorganism studied, exposing the cosmetic preparation to a standardized inoculum, followed by measuring changes in the number of microorganisms at set intervals, over a specified duration and at a specified temperature.

The results of the microbiological studies confirmed that all the products obtained were microbiologically clean.

The total count of aerobic mesophilic microorganisms, as well as the count of yeasts and molds, was less than 10 CFU. In the tested samples, no presence of pathogenic microorganisms such as Staphylococcus aureus (golden staph), Pseudomonas aeruginosa (blue pus bacillus), Escherichia coli (coliform bacteria), and Candida albicans (yeast) was detected.

## Claims

1. Method of producing a nanoemulgel composition with skin care and cosmetic properties, **characterized in that:**
- a pre-emulsion is created as a carrier for the active substance in the form of a pentacyclic triterpenoid compound, by dissolving the active substance in an amount of 0.1 to 0.5% by mass in oily emollients in an amount of 10 to 20% by mass at a temperature of 70-80 °C until a homogenous mixture is achieved; an emulsifier in an amount of 2 to 4% by mass is added, followed by adding a portion of the water phase with a preservative in an amount of 2.5 to 4.5% by mass, maintaining the temperature at 70-80 °C and mechanically stirring at a speed of 300 to 500 rpm during pre-emulsification for 5 to 15 minutes at a temperature of 20-25 °C;
- in the next stage, the prepared pre-emulsion undergoes a process of breaking down the particles of the active substance - the pentacyclic triterpenoid compound, into nanoparticles of equal to or smaller than 200 nm, through the application of ultrasound with a power of 20 to 40 W and an amplitude of 50 to 90 µm for a duration of 60 to 120 seconds, resulting in the formation of a nanoemulsion;
- in the final stage, a nanoemulgel is produced by introducing a separately prepared gelling matrix into the obtained nanoemulsion; this matrix consists of a portion of the water phase, dispersed with a gelling agent in an amount of 1 to 2% by mass, heating the system to a temperature of 40-50°C, adding a pH regulator q.s. to achieve a pH of 6, and simultaneously mechanically stirring at a speed of 400 to 600 rpm, until a homogenous nanoemulgel system is obtained.

2. Method according to claim 1, **characterized in that** the active substance is a pentacyclic triterpenoid compound selected from a group that includes ursolic acid, oleanolic acid, betulinic acid, betulin, boswellic acids (β-boswellic acid, acetyl-β-boswellic acid, 11-keto-β-boswellic acid, acetyl 11-keto-β-boswellic acid), α- and β-amyrin, lupeol.

3. Method according to claim 1, **characterized in that** ursolic acid in an amount of 0.1 to 0.5% by mass constitutes the active substance.

4. Method according to claim 1, **characterized in that** oleanolic acid in an amount of 0.1 to 0.5% by mass constitutes the active substance.

5. Method according to claim 1, **characterized in that** as oily emollients in an amount of 10 to 20% by mass, caprylic and capric triglycerides are used.

6. Method according to claim 1, **characterized in that** as an emulsifier in an amount of 2 to 4% by mass, polyglycerol-6 esters of olive oil combined with sodium stearoyl lactylate and cetearyl alcohol are used.

7. Method according to claim 1, **characterized in that** as a preservative in an amount of 2 to 4% by mass, an aqueous solution of glycerin, sodium levulinate, sodium anisate, and sodium benzoate is used.

8. Method according to claim 1, **characterized in that** as a gelling agent in an amount of 1 to 2% by mass, sodium hyaluronate is used.

9. Method according to claim 1, **characterized in that** the obtained nanoemulgel, as a base containing the active substance in the form of a pentacyclic triterpenoid compound in an amount of 0.1 to 0.5% by mass with reduced nanoparticle size equal to or smaller than 200 nm, is combined in a mass ratio ranging from 10:90 to 90:10 with a product in the form of cream, serum, or balm, containing the same repeated active substance in the form of a pentacyclic triterpenoid compound in an amount of 0.1 to 1.0% by mass in a form not subjected to particle size reduction of the active substance, obtaining a skin care and cosmetic composition.

10. Method according to claim 1, **characterized in that** the obtained nanoemulgel, as a base containing the active substance in the form of a pentacyclic triterpenoid compound in an amount of 0.1 to 0.5% by mass with reduced nanoparticle size equal to or smaller than 200 nm, is combined in a mass ratio ranging from 5:95 to 40:60 with a product in the form of an oleogel, or scalp preparation, containing the same repeated active substance in the form of a pentacyclic triterpenoid compound in an amount of 0.1 to 1.0% by mass in a form not subjected to particle size reduction of the active substance, obtaining a skin care and cosmetic composition.

11. Nanoemulgel composition with skin care and cosmetic properties for topical application, **characterized in that** it takes the form of a nanoemulgel and contains:
- as the active substance, a pentacyclic triterpenoid compound in an amount of 0.1 to 0.5% by mass, in the form of nanoparticles with a particle size reduced to equal to or smaller than 200 nm, selected from a group that includes ursolic acid, oleanolic acid, betulinic acid, betulin, boswellic acids (β-boswellic acid, acetyl-β-boswellic acid, 11-keto-β-boswellic acid, acetyl 11-keto-β-boswellic acid), α- and β-amyrin, lupeol;
- oily emollients in an amount of 10 to 20% by mass;
- emulsifier in an amount of 2 to 4% by mass;
- preservative in an amount of 2.5 to 4.5% by mass;
- pH regulator q.s;
- gelling agent in an amount of 1 to 2% by mass;.
- water up to 100%.

12. Composition according to claim 11, **characterized in that** the pentacyclic triterpenoid compound in an amount of 0.1-0.5% by mass constitutes ursolic acid or oleanolic acid.

13. Composition according to claim 11, **characterized in that** the oily emollients of the composition in an amount of 10 to 20% by mass consist of caprylic and capric triglycerides.

14. Composition according to claim 11, **characterized in that** the emulsifier in an amount of 2 to 4% by mass consists of polyglycerol-6 esters of olive oil combined with sodium stearoyl lactylate and cetearyl alcohol.

15. Composition according to claim 11, **characterized in that** the preservative in an amount of 2.5 to 4.5% by mass consists of an aqueous solution of glycerin, sodium levulinate, sodium anisate, and sodium benzoate.

16. Composition according to claim 11, **characterized in that** the gelling agent in an amount of 1 to 2% by mass consists of sodium hyaluronate.

17. Composition according to claim 11, **characterized in that** as a base nanoemulgel, it is combined in a mass ratio ranging from 10:90 to 90:10 with a product in the form of cream, serum, or balm, containing the same repeated active substance in the form of a triterpenic acid in an amount of 0.1 to 1.0% by mass in a form not subjected to particle size reduction.

18. Composition according to claim 11, **characterized in that** as a base nanoemulgel, it is combined in a mass ratio ranging from 5:95 to 40:60 with a product in the form of an oleogel, or scalp preparation, containing the same repeated active substance in the form of a triterpenic acid in an amount of 0.1 to 1.0% by mass in a form not subjected to particle size reduction.
